# EUROPÄISCHE PATENTSCHRIFT

(11) **EP 0 367 752 B1**
(45) Veröffentlichungstag und Bekanntmachung des Hinweises auf die Patenterteilung: **01.12.1993**
(21) Anmeldenummer: 89890261.4
(22) Anmeldetag: 05.10.1989
(51) Int. Cl.: A61B 5/00, A61M 5/172

(54) **Vorrichtung zur Bestimmung der Konzentration von zumindest einer in organischem Gewebe vorliegenden Substanz**
Device for determining the concentration of at least one substance in living tissue
Dispositif pour déterminer la concentration d'une ou plusieurs substances présentes dans le tissu vivant

(30) Priorität: 31.10.1988 AT 2682/88
(43) Veröffentlichungstag der Anmeldung: 09.05.1990
(73) Patentinhaber: AVL Medical Instruments AG, 8207 Schaffhausen (CH)
(72) Erfinder: Skrabal, Falko, Prof. Dr., A-8010 Graz (AT)
(74) Vertreter: Krause, Walter, Dr. Dipl.-Ing.

(56) Entgegenhaltungen:
- WO-A-88/05643
- US-A- 4 311 789
- US-A- 4 535 786

## Beschreibung

Die Erfindung betrifft eine Vorrichtung zur Bestimmung der Konzentration von zumindest einer in organischem Gewebe vorliegenden Substanz, vorzugsweise der Glucosekonzentration, mit einer ins Gewebe einbringbaren Subcutannadel, wobei eine Pump- und Saugeinrichtung zur Zufuhr einer Perfusionsflüssigkeit und zur Abfuhr nach deren teilweisen Equilibration mit dem Gewebe vorhanden ist und der Grad der Wechselwirkung mit dem Gewebe durch endogene oder exogene Markergrößen der Perfusionsflüssigkeit bestimmbar ist, sowie mit einer Analyseneinrichtung mit Meßeinrichtungen zur Erfassung der Konzentration der zu bestimmenden Substanz und der Markergrößen.

Im medizinischen Bereich besteht häufig die Notwendigkeit einer oftmaligen oder auch kontinuierlichen Analyse der Zusammensetzung der Körperflüssigkeiten, um Störungen der Homöostase zu erfassen und beseitigen zu können. Um häufiges Blutabnehmen unnotwendig zu machen, gibt es seit vielen Jahren Versuche, Sensoren im Körper von Patienten zu plazieren, um laufend Informationen zu erhalten.

Eine besonders gefragte Anwendung dabei ist die kontinuierliche Bestimmung der Glucosekonzentration im Organismus. Aus den US-PSen 4 221 567, 4 253 456 und 4 516 580 sind beispielsweise Verfahren und Vorrichtungen zur Dialyse bekannt, bei welchen zumindest eine Kanüle oder Katheter in den Blutstrom eingebracht wird. Über Dialysemembranen wird eine komplette Equilibration einer Meßflüssigkeit mit dem Blut erreicht, welche anschließend auf die interessierende Substanz, beispielsweise Glucose, analysiert wird. Bei den auf einer Seite von der Meßflüssigkeit und auf der anderen Seite von der Körperflüssigkeit, beispielsweise von Blut, beaufschlagten Membranen, welche für eine ausreichende Equilibration genügend groß sein müssen, kommt es jedoch bald zu Verunreinigungen bzw. zu einer Verstopfung der Membranen durch Körpersubstanzen, sodaß der Austausch der interessierenden Stoffe rasch behindert wird. Bei den in den Blutstrom eingebrachten Fremdkörpern, wie Kanülen und Kathetern, besteht zudem die ständige Gefahr der Infektion, der Thrombose oder der Embolie. Außerdem stehen für über längere Zeit aufrecht zu erhaltende Therapien nur wenige Blutgefäße zur Verfügung, welche bald thrombosieren, sodaß das Verfahren nicht fortgesetzt werden kann.

Dazu ist nun aus der WO88/05643 eine Vorrichtung der eingangs genannten Art bekanntgeworden, welche die oben angeführten Nachteile vermeidet. Eine als "Glucose-Pen" bezeichnete Ausführungsform weist hier ein annähernd schreibstiftförmig gestaltetes Gehäuse auf, das an seinem einen Ende eine Subcutannadel aufweist. Im Inneren des Gehäuses befindet sich eine Kolbenpumpe mit einem Reservoir für die Perfusionsflüssigkeit, aus welchem mit Hilfe des Kolbens sowohl das Perfusat durch Verdrängung aus dem Kolbenraum herausgepumpt als auch in den hinter dem Kolben freiwerdende Auffangbehälter angesaugt werden kann. Mit Hilfe einer Hebeleinrichtung kann über ein allenfalls notwendiges Getriebe der Kolben manuell bewegt werden und Perfusionsflüssigkeit mittels eines ersten Lumens in der Subcutannadel an die Öffnungen in deren Wand gebracht und gleichzeitig durch ein zweites, beispielsweise konzentrisch dazu angeordnetes Lumen durch den Sog im Auffangbehälter gesammelt werden. Möglichst knapp an der Subcutannadel ist dabei anschließend an die abführende Leitung eine Analyseneinrichtung mit einer Meßkapillare für die zu bestimmende Substanz und die Markergröße angebracht. Es kann somit für kurze Zeit, z.B. in der Größenordnung von ca. 1 Minute oder weniger, Flüssigkeit durch die Subcutannadel gepumpt und nach deren teilweisen Equilibration wieder gesammelt werden. Bereits nach kurzer Zeit kann über eine optische Anzeige oder auch eine akustische Anzeige die wahre Konzentration der interessierenden Substanz, beispielsweise des Blutzuckers, erfaßt werden, die von der Analyseneinheit aus der Konzentration der Markersubstanz und der interessierenden Substanz errechnet wird. Um während der erforderlichen Zeit der Gewebeperfusion eine gleichmäßige Flußrate zu erhalten, sind mehrere Vorgangsweisen denkbar. So kann z.B. über die Hebeleinrichtung deren Hebel während der Perfusionsperiode immer wieder betätigt wird, eine gleichmäßige Perfusionsrate erzielt werden. Der Hebel kann dazu, beispielsweise über eine Feder oder einen anderen Energiespeicher, immer wieder in seine Ausgangslage gebracht werden. Die Perfusionsrate während der Sammelperiode liegt dabei in der Größenordnung zwischen 1 und 10 µl/min. Nachteilig dabei ist lediglich der komplizierte Aufbau dieses "Glucose-Pen", wobei insbesondere die Herstellung der mehrlumigen Subcutannadel technisch schwierig und kostenintensiv ist.

Weiters ist aus der US-PS 4 535 786 ein Verfahren und eine Vorrichtung zur Analyse von Körperflüssigkeiten bekannt geworden, bei welchem eine einlumige Nadel verwendet wird, um eine Referenzflüssigkeit in einen Patienten zu infundieren, wobei die Referenzflüssigkeit an einem Sensor vorbeigeleitet wird, um diesen zu kalibrieren. Der Sensor besteht aus einer selektiven Elektrode und einer Referenzelektrode. Anschließend wird der Flüssigkeitsstrom umgekehrt, sodaß Körperflüssigkeit angesaugt wird und an die selektive Elektrode gelangt. Die Messung erfolgt zu einem Zeitpunkt, wo die Referenzelektrode noch immer mit der Referenzflüssigkeit in Kontakt steht.

Aufgabe der Erfindung ist es, eine Vorrichtung vorzuschlagen, welche ausgehend vom genannten Stand der Technik einfacher und kostengünstiger herstellbar ist, wobei die wesentlichen Vorteile der gattungsgemäßen Vorrichtung erhalten bleiben sollen.

Diese Aufgabe wird erfindungsgemäß dadurch gelöst, daß eine einlumige Subcutannadel vorgesehen ist, deren Lumen mit der Eingangsseite der Analyseneinrichtung verbindbar ist, daß die Subcutannadel neben der Frontöffnung zumindest eine weitere Öffnung oder schlitzförmige Erweiterung im Wandbereich aufweist, daß die Analyseneinrichtung ausgangsseitig über ein Mehrwegventil an die Pump- und Saugeinrichtung angeschlossen ist, sodaß die Pump- und Saugeinrichtung in einer ersten Stellung des Ventils mit der Subcutannadel und in einer zweiten Stellung mit einem Perfusionsbehälter in Verbindung steht, sowie daß die Strömungsrichtung der Perfusionsflüssigkeit im Lumen der Subcutannadel beim Wechsel vom Pump- zum Saugbetrieb umkehrbar ist. Mit dieser sehr einfachen Vorrichtung kann frisches Perfusat in einer Stellung des Ventils aus dem Perfusionsbehälter durch eine Saugbewegung der Pumpe entnommen und nach Umschalten des Ventils über die Subcutannadel mit einer Pumpbewegung in das Gewebe eingebracht werden. Mit einer anschließenden Saugbewegung bei gleicher Ventilstellung läßt sich das Perfusat wieder aus dem Körper gewinnen und der Meßvorrichtung zuführen. Nach dem Einstechen der Subcutannadel werden somit wenige µl Perfusat in die Subcutannadel gepumpt, wobei die Perfusionslösung die Analyseneinrichtung passiert und eine Nullpunktseichung vorgenommen werden kann. Nach teilweiser Equilibration des Perfusates mit dem an der Subcutannadel anstehenden Gewebe wird das Perfusat aus der Nadel gesaugt und wieder mit den Sensoren der Analyseneinrichtung in Kontakt gebracht, wobei die zu bestimmende Substanz und die Markergröße, z.B. die elektrische Leitfähigkeit, gemessen und aus diesen Werten die tatsächliche Konzentration, z.B. die Glucosekonzentration, errechnet wird. Zur Nullpunktseichung sollte die Perfusionsflüssigkeit im angeführten Beispiel im wesentlichen ionen- und glucosefrei sein.

Als weiterer Vorteil der erfindungsgemäßen Vorrichtung ist anzuführen, daß einlumige Subcutannadeln entsprechend dünner ausgeführt werden können, sodaß der Patient, z.B. bei der mehrmals täglich durchzuführenden Gewebezucker-Analyse, eine geringere Schmerzbelastung zu tragen hat.

Um die Austauschfläche der Subcutannadel zu vergrößern, können mehrere Öffnungen in mehreren Reihen an der Zirkumferenz der Nadel angebracht sein.

In einer Ausgestaltung der Erfindung ist vorgesehen, daß die Analyseneinrichtung an beiden Seiten eine konische Steckverbindung, aufweist, mit dem die Analyseneinrichtung auf der einen Seite mit der Subcutannadel und auf der anderen Seite über eine Verbindungsleitung mit der Pump- und Saugeinrichtung verbunden ist, wobei die Steckverbindung über lösbare Kontakte eine elektrische Verbindung zwischen Auswerteeinheit und Analyseneinrichtung herstellt. Die Steckverbindungen garantieren ein einfaches Wechseln der Nadel bzw. der Analyseneinrichtung.

Um dafür Sorge zu tragen, daß die teilweise equilibrierte Perfusionsflüssigkeit sich nicht mit der Perfusionsflüssigkeit in der Kolbenpumpe vermischen kann, ist in einer Weiterbildung der Erfindung vorgesehen, daß die Pump- und Saugeinrichtung in einer dritten Stellung des Mehrwegventils mit einer Öffnung zur Zufuhr von Außenluft in Verbindung steht. Die über das Ventil einsaugbare Luftblase trennt dann die beiden Flüssigkeitssäulen.

In einer Ausgestaltung der Erfindung ist vorgesehen, daß die Pump- und Saugeinrichtung eine Kolbenpumpe aufweist, sowie daß für den Saughub ein während des Pumphubes aufgeladener Energiespeicher vorgesehen ist. Bei einer vorzugsweise von Hand betätigbaren Kolbenpumpe muß der Patient lediglich nach dem Einstechen eine Hebeleinrichtung drücken und später loslassen, wodurch der Analysiervorgang automatisch abläuft. Als Energiespeicher dient beispielsweise eine Feder. Durch kleine Pumpbewegungen während des Equilibriervorganges kann die Strömungsrichtung des Perfusates im Lumen der Subcutannadel mehrfach umgekehrt werden, wodurch ein höherer Equilibrationsgrad erreicht wird.

Eine vorteilhafte Weiterbildung der Erfindung liegt darin, daß im Pumpenantrieb ein Mechanismus vorgesehen ist, welcher zwischen Pump- und Saughub, in der Equilibrierphase der Perfusionsflüssigkeit, für kleine Pump- und Saugbewegungen des Kolbens der Kolbenpumpe sorgt. Damit werden die Pumpbewegungen in der Equilibrationsphase von einem Mechanismus ausgeführt, welchem die erforderliche Energie ebenfalls über die den Pumphub auslösende Hebeleinrichtung zugeführt werden kann.

Es kann in einer erfindungsgemäßen Weiterbildung auch vorgesehen sein, daß zumindest ein weiterer Behälter für ein zu verabreichendes Medikament vorgesehen ist, sowie daß die Pump- und Saugeinrichtung in zumindest einer weiteren Stellung des Mehrwegventils mit je einem Behälter für ein Medikament in Verbindung steht.

Erfindungsgemäß kann dabei ein Behälter für ein rasch wirksames Insulin, ein Behälter für lange wirkendes Insulin und ein Behälter für ein blutzuckersteigerndes Hormon vorgesehen sein.

Eine Weiterbildung nach der Erfindung sieht vor, daß der Perfusionsbehälter und die Behälter für Medikamente leicht verformbare Beutel aus Kunststoff sind und gemeinsam mit dem Mehrwegventil und den entsprechenden Verbindungsleitungen eine austauschbare Einheit bilden. Damit kann dann die aufgrund des Meßwertes der Konzentration der zu bestimmenden Substanzen erkennbare, erforderliche Medikamentenmenge ohne weitere Stichbelastung des Patienten über die bereits eingeführte Subcutannadel appliziert werden. Nachdem der Perfusionsbehälter bzw. der Behälter für das Medikament entleert ist, können diese mitsamt dem Mehrwegventil und den erforderlichen Verbindungsleitungen als Einheit gegen eine neue ausgewechselt werden.

Dabei ist in einer Weiterbildung der Erfindung vorgesehen, daß die austauschbare Einheit über eine druckdichte Verbindung, vorzugsweise über ein Durchstichseptum oder eine O-Ringdichtung mit der Pump- und Saugeinrichtung in Verbindung steht, wobei der Innenraum des zwischen dem Mehrwegventil und der druckdichten Verbindung liegenden Leitungsabschnittes der Verbindungsleitung dem Volumen zumindest eines Saughubes entspricht. Der zwischen Septum bzw. O-Ring-Dichtstelle und Mehrwegventil liegende Leitungsabschnitt kann aufgrund seines Innenvolumens einen Saughub zur Gänze aufnehmen, wodurch die anschließende Pump- und Saugeinrichtung nicht kontaminiert wird. Das benötigte Volumen kann entweder durch eine aufgerollte Kapillarleitung oder durch entsprechend geräumige Erweiterungen in der Leitung realisiert werden, wobei eine Kapillarleitung allerdings den Vorteil hat, daß sie ein Funktionieren der Vorrichtung völlig lageunabhängig gewährleistet, da sich die Flüssigkeitssäule in der Kapillare nicht unabsichtlich mit Luft durchmischen kann.

Um eine Kontamination der Pump- und Saugeinrichtung sicher zu vermeiden, ist entsprechend einer Weiterbildung der Erfindung vorgesehen, daß die austauschbare Einheit zwischen der druckdichten Verbindung und dem Mehrwegventil eine durch den anliegenden Druck elastisch verformbare Membran aufweist. Es ist auch möglich, anstelle der Membran ein gasduchlässiges Bakterienfilter zu verwenden, welches für Flüssigkeiten undurchlässig ist.

Falls die Vorrichtung für längere Zeit am Patienten verbleiben soll, ist es von Vorteil, wenn zwischen der konischen Steckverbindung zum Mehrwegventil und der Analyseneinrichtung eine verformbare Verbindungsleitung und eine elektrische Verbindung vorgesehen sind. Die Subcutannadel samt Analyseneinrichtung bildet hier eine Einheit, welche mit dein die übrigen Komponenten der Vorrichtung beinhaltenden Gehäuse beweglich verbunden ist.

Für Fälle, wo nicht das gesamte verbrauchte Perfusat in das Gewebe abgeleitet werden kann, ist es vorteilhaft, wenn ein Auffangbehälter für die Perfusionsflüssigkeit vorgesehen ist, welcher über eine weitere Stellung des Mehrwegventils mit der Pump- und Saugeinrichtung in Verbindung steht.

Die Pump- und Saugeinrichtung kann somit ohne Reinigung und Wartung für lange Zeit im Gehäuse der Vorrichtung verbleiben, womit erfindungsgemäß eine von einem Mikroprozessor gesteuerte Präzisionspumpe verwendet werden kann, welche den großen Anforderungen im Hinblick auf Sicherheit und Reproduzierbarkeit gerecht ist. Der Mikroprozessor steuert gleichzeitig auch das Mehrwegventil, wobei über ein geeignetes Display für den Benutzer lediglich Hinweise, wie "Nadel aufstecken", "Nadel einstechen", "Nadel entfernen", etc. aufscheinen.

Die Verabreichung des langsam wirkenden Insulins, die des rasch wirkenden Insulins sowie der blutzuckersteigernden Substanz kann vorteilhafterweise über den Mikroprozessor der Auswerteeinheit gesteuert werden. Dabei ist es von Vorteil, wenn die Gewebszuckerwerte und die abgegebenen Insulinmengen in einem Speicher über möglichst lange Zeit gespeichert werden, damit die errechneten Algorithmen für die Insulingabe durch einen Lerneffekt im Laufe der Zeit verbessert werden können. Es ist auch möglich, die Pump- bzw. Saugleistung der Pump- und Saugeinrichtung mit Hilfe des Mikroprozessors so zu steuern, daß die Markergröße, beispielsweise die Leitfähigkeit, im wesentlichen konstant bleibt. Nach Erreichen einer wünschenswerten Equilibration zwischen 5 bis 20 % der Gewebekonzentration kann die Saugeinrichtung eventuell abgeschaltet werden.

Weiters ist erfindungsgemäß vorgesehen, daß zur raschen Umkehr der der Strömungsrichtung der Perfusionsflüssigkeit ein während des Pumpbetriebes der Präzisionspumpe mit Unterdruck beaufschlagter Pumpenraum im Saugbetrieb in einer weiteren Stellung des Ventiles über eine Verbindungsleitung mit der Subcutannadel verbindbar ist.

Vor allem dann, wenn die Analyseneinrichtung über eine flexible Verbindungsleitung mit dem Gehäuse der Vorrichtung verbunden ist und lange Signalwege vorhanden sind, ist erfindungsgemäß vorgesehen, daß in der Analyseneinrichtung ein Vorverstärker eingebaut ist.

In allen Ausführungsvarianten kann die Analyseneinrichtung zur besseren Durchmischung der zu messenden Flüssigkeit eingangsseitig eine Veränderung des Strömungsquerschnittes, z.B. in Form einer Noppe, aufweisen, welche in den Perfusatstrom ragt und die Perfusionsflüssigkeit verwirbelt.

Zur besseren Absicherung der Meßergebnisse kann von der Analyseneinrichtung zuerst die Konzentration der Markergröße, anschließend die Konzentration der interessierenden Substanz und anschließend zumindest noch einmal die Konzentration der Markergröße bestimmt werden, bzw. in zwei unabhängigen Meßkreisen die Markergröße und die Konzentration der interessierenden Substanz gleichzeitig und kontinuierlich gemessen werden, und aus diesen Messungen die tatsächliche Konzentration der interessierenden Substanz, z.B. des Gewebezuckers, errechnet werden.

Schließlich ist erfindungsgemäß vorgesehen, daß in der Analyseneinrichtung ein kombinierter Ionen- oder Leitfähigkeitssensor sowie ein Glucosesensor vorgesehen sind, wobei die Analyseneinrichtung mit den kombinierten Sensoren ein Füllvolumen von ca. 10 µl oder weniger aufweist.

Die Erfindung wird im folgenden anhand von Zeichnungen näher erläutert. Es zeigen:
- Figur 1: eine Vorrichtung nach der Erfindung in schematischer Darstellung,
- Figur 2: eine Ausführungsvariante nach Fig. 1,
- Figur 3a und 3b: Ausführungsvarianten der Vorrichtung im Detail und
- Figur 4: eine Meßkurve.

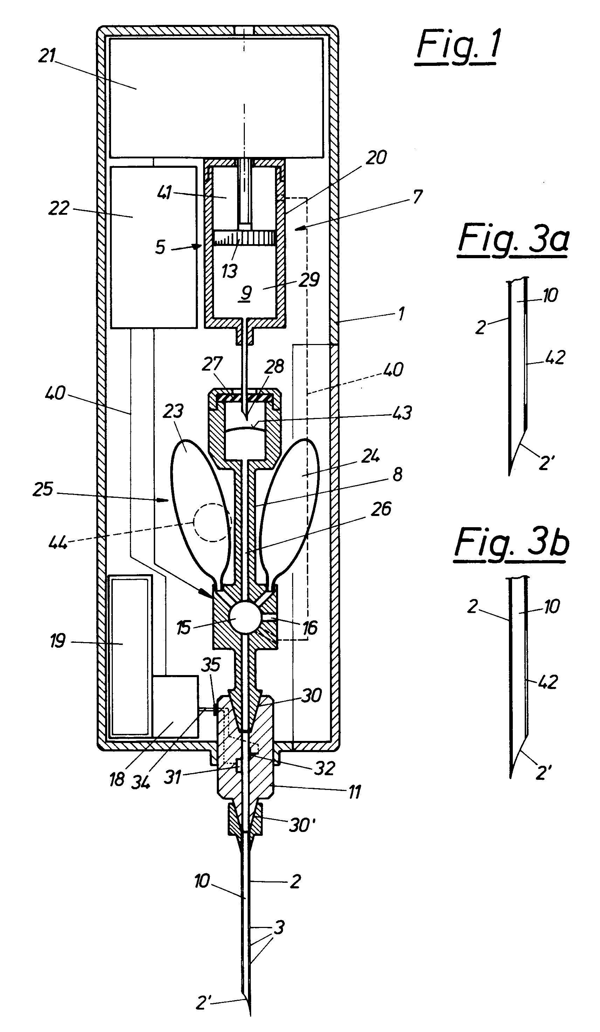

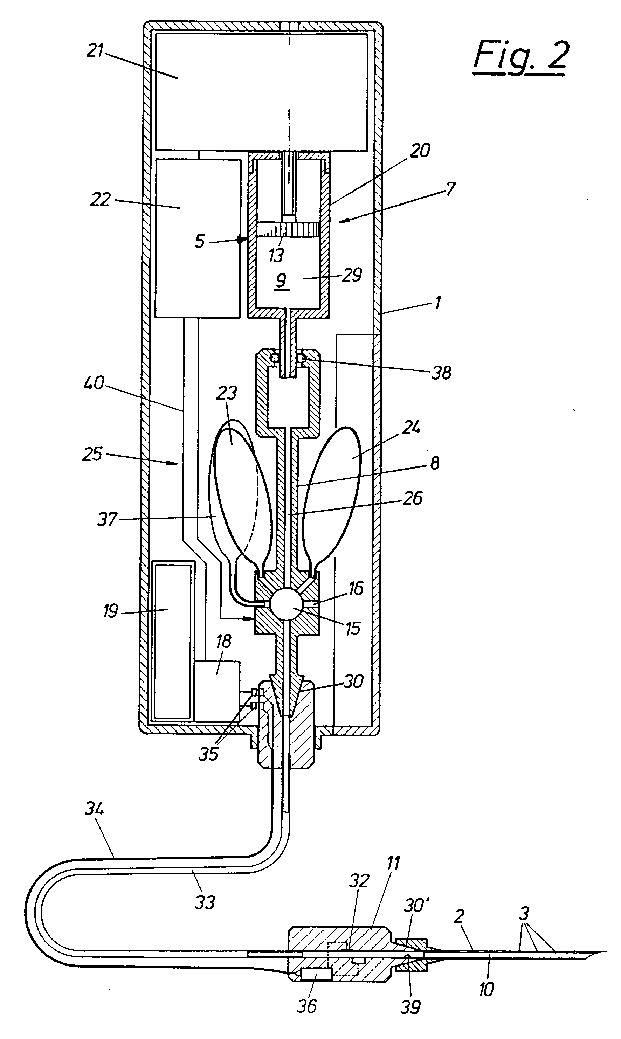

Die in Fig. 1 dargestellte Vorrichtung weist eine an einem Gehäuse 1 befestigte, einlumige Subcutannadel 2 mit einer Frontöffnung 2' auf, in deren Wand Öffnungen 3 zum umliegenden Gewebe 4 angeordnet sind. Im Gehäuse 1 ist eine z.B. aus einer Kolbenpumpe 5 bestehende Pump- und Saugeinrichtung 7 zur Förderung der Perfusionsflüssigkeit angeordnet. In der Verbindungsleitung 8, zwischen dem Kolbenraum 9 der Kolbenpumpe 5 und dem Lumen 10 der Subcutannadel 2, ist direkt dem Ausgang der Subcutannadel 2 eine Analyseneinrichtung 11 mit hier nicht näher dargestellten Sensoren zur Erfassung der Konzentration der zu messenden Substanz und der Markergrößen der Perfusionsflüssigkeit zugeordnet.

Der in Pumprichtung bewegte Kolben 13 der Kolbenpumpe 5 verdrängt einige Mikroliter Perfusionsflüssigkeit aus dem Kolbenraum 9, wobei das Perfusat auf seinem Weg zur Subcutannadel 2 die Analyseneinrichtung 11 zur Nullpunktskalibrierung passiert. Durch kleine Pumpbewegungen kann nun eventuell zusätzlich für eine mehrmalige Umkehr der Strömungsrichtung des Perfusates im Lumen 10 der Subcutannadel 2 gesorgt werden, wodurch eine ausreichende Equilibration erreicht wird. Durch Umkehr der Pumprichtung wird der Saughub bewerkstelligt und das teilweise equilibrierte Perfusat wieder mit der Analyseneinrichtung 11 in Kontakt gebracht und gemessen.

Über eine an die Analyseneinrichtung 11 angeschlossene Auswerteeinheit 18 und ein Display 19 gelangen die gemessenen Werte zur Anzeige.

Die Pump- und Saugeinrichtung 7 kann auch durch eine Präzisionspumpe 20 realisiert werden, deren Kolben 13 von einem Schrittmotor 21 bewegt wird. Die einzelnen Saug- und Pumpbewegungen der Präzisionspumpe 20, sowie die jeweils notwendigen Ventilstellungen eines Mehrwegventils 15 werden von einem Mikroprozessor 22 gesteuert. Der Perfusatbehälter 23 und ein Behälter 24 für das zu verabreichende Medikament bilden zusammen mit dem Mehrwegventil 15 eine auswechselbare Einheit 25, welche über den Leitungsabschnitt 26 der Verbindungsleitung 8 mit der Präzisionspumpe 20 verbunden ist.

Bei Verwendung von mehr als einem Medikament zur Korrektur der festgestellten Störung, könnten auch mehrere Medikamentenbehälter 24 angebracht sein. So könnte z.B. im Falle der Zuckermessung ein Behälter für ein rasch wirksames Insulin, ein anderer Medikamentenbehälter für ein lang wirkendes Insulin, und ein weiterer Medikamentenbehälter zur Hebung des Blutzuckers, z.B. Glucagon, vorhanden sein.

Der Leitungsabschnitt 26 der Verbindungsleitung 8 ist mit einem Durchstichseptum 27 verschlossen, über welches die Einheit 25 an den mit einer Nadel 28 versehenen Kolbenraum 9 der Präzisionspumpe 20 anschließt. Die Präzisionspumpe kann dabei ähnlich aufgebaut sein, wie sie von Mikroliterpipetten bekannt ist, z.B. ein Kolben, der mit einem oder mehreren O-Ringen gegen das Gehäuse abgedichtet ist. Um die Präzisionspumpe 20 vor Kontaminationen zu schützen, kann vor dem Septum 27 eine elastisch verformbare Membran 43 angeordnet sein.

Es ist auch möglich andere Pumpen, wie z.B. eine Rollenpumpe, die einen verformbaren Schlauch komprimiert, eine Membranpumpe, oder einen Druckbehälter mit einem komprimierten Medium, zu verwenden. Ein günstiger Platz für die Anordnung einer Rollenpumpe 44 wäre beispielsweise an einem verformbar ausgebildeten Leitungsabschnitt 26 gegeben, da hier Saug- und Pumpvorgänge durchgeführt werden können, ohne unbeabsichtigt Flüssigkeit in die Nadel 2 zu pumpen, bzw. andere Fehlfunktionen herbeizuführen.

Der Innenraum des Leitungsabschnittes 26 zwischen dem Ventil 15 und dem Septum 27 ist so dimensioniert, daß das Volumen eines Saughubes der Präzisionspumpe aufgenommen werden kann, ohne die Präzisionspumpe 20 mit ihren im Gehäuse 1 verbleibenden Teilen mit einem der zu fördernden Medien zu kontaminieren. Mit Hilfe des Ventils 15 kann die Präzisionspumpe 20 in definierten Arbeitsstellungen mit der Subcutannadel 2, mit dem Perfusatbehälter 23, sowie mit den Behältern 24 für Medikamente verbunden werden. Um zu verhindern, daß sich bereits equilibriertes Perfusat und frisches Perfusat vermischen, kann in einer weiteren Stellung des Mehrwegventils 15 über eine Öffnung 16 im Gehäuse 1 eine Luftblase zur Trennung der unterschiedlichen Flüssigkeiten eingesaugt werden kann.

Weiters ist es auch möglich, die Subcutannadel 2 über das Ventil 15 und eine in Fig. 1 strichliert angedeutete Verbindungsleitung 40 mit dem hinter dem Kolben 13 liegenden Pumpenraum 41 der Pumpe 20 zu verbinden. Der während des Pumpenhubes im Pumpenraum 41 erzeugte Unterdruck sorgt dann für eine rasche Umkehr der Strömungsrichtung des Perfusates in der Subcutannadel 2.

Die Analyseneinrichtung 11 mit dem Ionen- oder Leitfähigkeitssensor 31 und dem Glucosesensor 32 steht mit der auswechselbaren Einheit 25 über eine konische Steckverbindung 30, vorzugsweise über einen Luerverschluß, in Verbindung. Mit einer konischen Steckverbindung 30' gleicher Bauart ist die Subcutannadel 2 mit der Analyseneinrichtung 11 verbunden.

Dabei kann automatisch auch eine elektrische Verbindung 34 zwischen Analysenvorrichtung 11 mit dem Glucosesensor 32 und dem Leitfähigkeitsmesser 31 einerseits und der Auswerteeinheit 18 bewerkstelligt werden, wobei es sich z.B. um eine Steckverbindung oder Gleitkontakte 35 handeln könnte. Der Mikroprozessor 22 steht über eine Leitung 40 mit der Auswerteeinheit 18 in Verbindung.

Zumindest einem Behälter 24 für das zu verabreichende Medikament (Insulin) oder einem separaten Behälter kann eine geringe Menge der zu messenden Substanz (Glucose) sowie einer Markersubstanz (z.B. Natriumchlorid) zugesetzt sein, wobei deren Konzentration im Bereich der üblicherweise beobachteten Konzentrationen liegt.

Fig. 2 zeigt eine weitere mögliche Ausführungsform der Vorrichtung, die speziell dafür gedacht ist, als "künstliches Pankreas" länger mit dem Patienten in Verbindung zu stehen. Diese Ausführungsform unterscheidet sich im wesentlichen nur insoferne von der in Fig. 1 gezeigten, als zwischen der Steckverbindung 30, die die Verbindung zum Mehrwegventil 15 herstellt einerseits und der Analyseneinrichtung 11 andererseits eine flexible Verbindungsleitung 33 besteht, sodaß die Nadel 2, die z.B. in diesem Fall aus weichem, atraumatischem Material bestehen kann, leicht im subcutanene Gewebe angebracht werden kann, und die übrige Vorrichtung an anderer Stelle am Körper befestigt wird. Dies hat den Vorteil, daß Lageänderungen der Vorrichtung keinen Zug oder Druck auf die Nadel 2 ausüben können. Die Analyseneinrichtung 11 soll möglichst knapp an der Subcutannadel 2 angebracht sein, wobei die Steckverbindung 30' zwischen Analyseneinrichtung 11 und Nadel 2 einen Austausch der Analyseneinrichtung 11 ermöglicht, und die Nadel 2 trotzdem im Gewebe verbleiben kann. Andernfalls könnte die Analyseneinrichtung 11 natürlich auch direkt mit der Nadel 2 verbunden sein. Neben der die Flüssigkeiten transportierenden Verbindungsleitung 33 muß auch eine elektrische Verbindung 34 zur Auswerteeinheit 18 vorhanden sein, beispielsweise elektrische Kontakte 35, z.B. eine Steckverbindung oder Gleitkontakte, die gleichzeitig mit dem Anschluß der Steckverbindung 30 geschlossen werden. Wenn die Signale von der Analyseneinrichtung 11 oft sehr schwach und störungsanfällig sind, kann in der Analyseneinrichtung 11 auch ein Vorverstärker 36 untergebracht sein. Zur besseren Durchmischung des Perfusates vor der Messung ist am Eingang der Analyseneinrichtung 11 die Noppe 39 zur Veränderung des Strömungsquerschnittes angeordnet.

Für den längeren Verbleib der Vorrichtung im Körper bei Verwendung z.B. als künstliches Pankreas oder als anderes "closed feedback" System kann es günstig sein, auch einen zusätzlichen Auffangbehälter 37 zu schaffen, der nach der Messung das verbrauchte Perfusat aufnimmt, damit es nicht in den Körper gepumpt werden muß. Auch dieser Auffangbehälter 37 kann aus leicht verformbarem Material hergestellt sein. Weiters gezeigt ist mit 38 die O-Ringdichtung, die statt des Durchstichseptums 27 und der Nadel 28 verwendet werden kann, wobei durch Anbringen einer Verschlußkappe, die nicht gezeigt ist, vor dem Einsetzen der austauschbaren Einheit 25 für Sterilität gesorgt werden kann. Diese Verschlußkappe wird erst unmittelbar vor dem Einsetzen der Einheit 25 in die Vorrichtung abgenommen.

Wie im Detail in Fig. 3a dargestellt, kann die Subcutannadel 2 im Bereich der Frontöffnung 2' eine schlitzförmige Erweiterung 42 aufweisen, wodurch ebenfalls die Austauschfläche der Subcutannadel 2 vergrößert wird. Entsprechend Fig. 3b kann die Erweiterung auch direkt an die Frontöffnung 2' anschließen.

Im Betrieb der erfindungsgemäßen Vorrichtung sind folgende, im wesentlichen automatisch ablaufende Schritte denkbar:
1. Bevor die Subcutannadel 2 in den Körper eingebracht wird, wird durch die Analyseneinrichtung 11 eine Lösung geschickt, die ca. eine Menge der zu bestimmenden Substanz und der Markersubstanz enthält, wie sie dann bei der aktuellen Messung erwartet wird, womit die Steilheit der Sensoren ermittelt werden kann. Beispielsweise kann dazu der Medikamentenlösung, z.B. Insulin, die zu bestimmende Substanz , wie z.B. Glucose und die Markersubstanz, wie z.B. Natriumchlorid, zugesetzt werden. Anschließend wird über das Ventil 15 Luft angesaugt und die Verbindungsleitung 8, sowie die Analyseneinrichtung 11 durch Ausstoßen von Luft gereinigt. Anschließend wird ein Perfusat, das frei von zu bestimmender Substanz und Markersubstanz ist, durch die Analyseneinrichtung 11 gepumpt um den Nullpunkt nachzueichen.
2. Nach dem Aufstecken und Einstechen der Subcutannadel wird das Perfusat in die Nadel gepumpt und sehr rasch an die Subcutannadel ein Unterdruck angelegt, um das in die Nadel gepumpte Perfusat wieder zu gewinnen, bevor es im Körper versickern kann. Eventuell kann zusätzlich durch kleine Pumpbewegungen im Lumen der Subcutannadel das Perfusat hin und her bewegt werden bis eine teilweise Equilibration erreicht ist. Im Anschließenden Saughub wird die Konzentration der interessierenden Substanz, z.B. Glucose und die Markergröße, z.B. die Leitfähigkeit als Maß für den Grad der Equilibration gemessen.
3. Das inerte Perfusat kann anschließend in den Körper oder wie in Fig. 2 dargelegt, in einen eigenen Auffangbehälter gepumpt werden.
4. Anschließend wird das Mehrwegventil umgeschaltet und die erforderliche Menge des Medikamentes, z.B. Insulin oder auch eine blutzuckersteigernde Substanz, wie z.B. Glucagon in das Gewebe gepumpt. Dabei kann auch vom Mikroprozessor 19 aufgrund der erhobenen Meßwerte die optimale Menge des Medikamentes ohne Zutun des Patienten errechnet werden, der Patient kann dann eventuell die vorgeschlagene Menge des Medikamentes durch Tastendruck bestätigen.
5. Nach der Applikation der Medikamentes wird über das Ventil 15 nochmals eine Verbindung zur Außenluft oder zum Perfusionsbehälter hergestellt und der Rest des Medikamentes mit der Perfusionsflüssigkeit oder mit Luft aus der Subcutannadel in das Gewebe gespült.
6. Bei Anwendung der Vorrichtung als künstliches "Pankreas" kann die Nadel im Körper verbleiben und der Meßvorgang und die Insulinapplikation kann in geeigneten Intervallen automatisch wiederholt werden, wobei die Nadel durch intermittierende Spülung mit der Perfusionslösung frei von Verstopfungen gehalten werden kann und der Perfusionslösung auch gerinnungshemmende Stoffe zugesetzt sein können.

In der Praxis haben sich folgende Volumina, Zeitabläufe und Drucke bewährt, um möglichst rasch eine ausreichende Equilibration der Perfusionsflüssigkeit mit dem Gewebe zu erreichen:
- Injektion von ca. 20 Mikroliter bis 150 Mikroliter (am besten ca. 50 bis 100 Mikroliter) Perfusionsflüssigkeit durch eine Subcutannadel mit einem äußeren Querschnitt von ca, 0,4 mm und einer Länge zwischen 10 und 20 mm innerhalb ca. 1 Sekunde.
- Sofortiges Anlegen eines Unterdruckes zwischen 200 mbar und 700 mbar, vorzugsweise 400 mbar (bzw. eines Absolutdruckes zwischen 300 mbar und 800 mbar, vorzugsweise 600 mbar).
- Innerhalb von 20 Sekunden kommt es innerhalb eines Sensors mit einer Füllungskapazität von ca. 10 Mikroliter zu einer Equilibration zwischen 5 und 20 %, wobei das Ansaugen von Luft durch die Wahl des richtigen Druckes zu vermeiden ist.

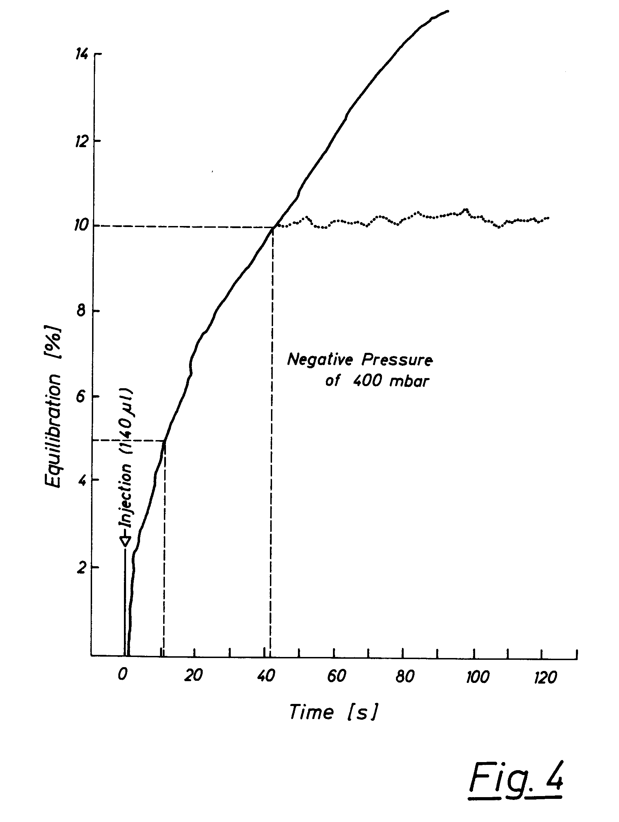

Fig. 4 zeigt den annähernd exponentiellen Verlauf der Equilibration am Sensor, wobei die beobachtete mathematische Funktion der Berechnung der tatsächlichen Konzentration der zu messenden Substanz dient. Die strichlierten Linien zeigen die nach 10 bzw. 40 Sekunden erreichte Equilibration von 5 % bzw. 10 % der Gewebeflüssigkeit. Durch Steuerung der Pump- und Saugeinrichtung kann der Grad der Equilibration, z.B. wie durch die punktierte Linie angedeutet, konstant gehalten werden.

## Patentansprüche

1. Vorrichtung zur Bestimmung der Konzentration von zumindest einer in organischem Gewebe vorliegenden Substanz, vorzugsweise der Glucosekonzentration, mit einer ins Gewebe einbringbaren Subcutannadel (2), wobei eine Pump- und Saugeinrichtung (7) zur Zufuhr einer Perfusionsflüssigkeit und zur Abfuhr nach deren teilweisen Equilibration mit dem Gewebe vorhanden ist und der Grad der Wechselwirkung mit dem Gewebe durch endogene oder exogene Markergrößen der Perfusionsflüssigkeit bestimmbar ist, sowie mit einer Analyseneinrichtung (11) mit Meßeinrichtungen zur Erfassung der Konzentration der zu bestimmenden Substanz und der Markergrößen, **dadurch gekennzeichnet**, daß eine einlumige Subcutannadel (2) vorgesehen ist, deren Lumen (10) mit der Eingangsseite der Analyseneinrichtung (11) verbindbar ist, daß die Subcutannadel (2) neben der Frontöffnung (2') zumindest eine weitere Öffnung (3) oder schlitzförmige Erweiterung (42) im Wandbereich aufweist, daß die Analyseneinrichtung (11) ausgangsseitig über ein Mehrwegventil (15) an die Pump- und Saugeinrichtung (7) angeschlossen ist, sodaß die Pump- und Saugeinrichtung (7) in einer ersten Stellung des Ventils (15) mit der Subcutannadel (2) und in einer zweiten Stellung mit einem Perfusionsbehälter (23) in Verbindung steht, sowie daß die Strömungsrichtung der Perfusionsflüssigkeit im Lumen (10) der Subcutannadel (2) beim Wechsel von, Pump- zum Saugbetrieb umkehrbar ist.

2. Vorrichtung nach Anspruch 1 bis, **dadurch gekennzeichnet**, daß die Analyseneinrichtung (11) an beiden Seiten eine konische Steckverbindung (30,30'), aufweist, mit dem die Analyseneinrichtung (11) auf der einen Seite mit der Subcutannadel (2) und auf der anderen Seite über eine Verbindungsleitung (8) mit der Pump- und Saugeinrichtung (7) verbunden ist, wobei die Steckverbindung (30 über lösbare Kontakte (35) eine elektrische Verbindung (34) zwischen Auswerteeinheit (18) und Analyseneinrichtung (11) herstellt.

3. Vorrichtung nach Anspruch 1 oder 2, **dadurch gekennzeichnet**, daß die Pump- und Saugeinrichtung (7) in einer dritten Stellung des Mehrwegventils (15) mit einer Öffnung (16) zur Zufuhr von Außenluft in Verbindung steht.

4. Vorrichtung nach einem der Ansprüche 1 bis 3, **dadurch gekennzeichnet**, daß die Pump- und Saugeinrichtung (7) eine Kolbenpumpe (5) aufweist, sowie daß für den Saughub ein während des Pumphubes aufgeladener Energiespeicher (14), vorgesehen ist.

5. Vorrichtung nach Anspruch 4, **dadurch gekennzeichnet**, daß im Pumpenantrieb ein Mechanismus vorgesehen ist, welcher zwischen Pump- und Saughub, in der Equilibrierphase der Perfusionsflüssigkeit, für kleine Pump- and Saugbewegungen des Kolbens (13) der Kolbenpumpe (5) sorgt.

6. Vorrichtung nach Anspruch 3, **dadurch gekennzeichnet**, daß zumindest ein weiterer Behälter (24) für ein zu verabreichendes Medikament vorgesehen ist, sowie daß die Pump- und Saugeinrichtung in zumindest einer weiteren Stellung des Mehrwegventils (15) mit je einem Behälter (24) für ein Medikament in Verbindung steht.

7. Vorrichtung nach Anspruch 6, **dadurch gekennzeichnet**, daß ein Behälter (24) für ein rasch wirksames Insulin, ein Behälter für lange wirkendes Insulin und ein Behälter für ein blutzuckersteigerndes Hormon vorgesehen ist.

8. Vorrichtung nach Anspruch 6 oder 7 **dadurch gekennzeichnet**, daß der Perfusionsbehälter (23) und die Behälter (24) für Medikamente leicht verformbare Beutel aus Kunnststoff sind und gemeinsam mit dem Mehrwegventil (15) und den entsprechenden Verbindungsleitungen (8, 26) eine austauschbare Einheit (25) bilden.

9. Vorrichtung nach Anspruch 8, **dadurch gekennzeichnet**, daß die austauschbare Einheit (25) über eine druckdichte Verbindung, vorzugsweise über ein Durchstichseptum (27) oder eine O-Ringdichttung (38) mit der Pump- und Saugeinrichtung (7) in Verbindung steht, wobei der Innenraum des zwischen dem Mehrwegventil (15) und der druckdichten Verbindung (27; 38) liegenden Leitungsabschnittes (26) der Verbindungsleitung (8) dem Volumen zumindest eines Saughubes entspricht.

10. Vorrichtung nach Anspruch 9, **dadurch gekennzeichnet**, daß die austauschbare Einheit (25) zwischen der druckdichten Verbindung (27; 38) und dem Mehrwegventil (15) eine durch den anliegenden Druck elastisch verformbare Membran (43) aufweist.

11. Vorrichtung nach Anspruch 2, **dadurch gekennzeichnet**, daß zwischen der konischen Steckverbindung (30) zum Mehrwegventil (15) und der Analyseneinrichtung (11) eine verformbare Verbindungsleitung (33) und eine elektrische Verbindung (34) vorgesehen sind.

12. Vorrichtung nach Anspruch 6, **dadurch gekennzeichnet**, daß ein Auffangbehälter (37) für die Perfusionsflüssigkeit vorgesehen ist, welcher über eine weitere Stellung des Mehrwegventils (15) mit der Pump- und Saugeinrichtung (7) in Verbindung steht.

13. Vorrichtung nach einem der Ansprüche 1 bis 3, **dadurch gekennzeichnet**, daß die Pump- und Saugeinrichtung (7) eine von einen Mikroprozessor (22) gesteuerte Präzisionspumpe (20) ist.

14. Vorrichtung nach Anspruch 13, **dadurch gekennzeichnet**, daß die Präzisionspumpe (20) eine Pumpleistung von 10 bis 200 µl/s aufweist und im Saugbetrieb innerhalb einer Sekunde einen Unterdruck von 200 bis 700 mbar erzeugt.

15. Vorrichtung nach Anspruch 13 oder 14, **dadurch gekennzeichnet,** daß zur raschen Umkehr der der Strömungsrichtung der Perfusionsflüssigkeit ein während des Pumpbetriebes der Präzisionspumpe (20) mit Unterdruck beaufschlagter Pumpenraum (41) im Saugbetrieb in einer weiteren Stellung des Ventiles (15) über eine Verbindungsleitung (40) mit der Subcutannadel (2) verbindbar ist.

16. Vorrichtung nach Anspruch 1, **dadurch gekennzeichnet**, daß in der Analyseneinrichtung (11) ein Ionen- oder Leitfähigkeitssensor (31) sowie ein Glucosesensor (32) vorgesehen sind.

17. Vorrichtung nach Anspruch 1, **dadurch gekennzeichnet**, daß in der Analyseneinrichtung (11) ein Vorverstärker (36) eingebaut ist.

18. Vorrichtung nach Anspruch 1, **dadurch gekennzeichnet**, daß die Analyseneinrichtung (11) eingangsseitig eine Veränderung des Strömungsquerschnittes in Form einer Noppe (39) aufweist.

19. Vorrichtung nach einem der Ansprüche 16 bis 19, **dadurch gekennzeichnet**, daß die Analyseneinrichtung (11) mit den kombinierten Sensoren (31, 32) ein Füllvolumen von ca. 10 µl aufweist.

## Claims

1. Apparatus for determining the concentration of at least one substance present in organic tissue, preferably the glucose concentration, with a subcutaneous needle (2) for introduction into the tissue, a pumping and suction device (7) being present for introducing a perfusion fluid and for removal of it after partial equilibration with the tissue and the degree of interaction with the tissue being determined by endogenic or exogenic marker magnitudes of the perfusion fluid, and with an analysing device (11) with measuring devices for obtaining the concentration of the substance to be determined and the marker magnitude, characterised in that a single-lumen subcutaneous needle (2) is provided, of which the lumen (10) can be connected to the input side of the analysing device (11), that the subcutaneous needle (2) has in addition to the front opening (2') at least one further opening (3) or slot-shaped widening (42), that the analysing device (11) is connected at its output side through a multi-way valve (15) to the pumping and suction device (7) so that the pumping and suction device (7) is in communication in a first position of the valve (15) with the subcutaneous needle (2) and in a second position with a perfusion reservoir (23), and that the direction of flow of the perfusion fluid in the lumen (10) of the subcutaneous needle (2) is reversible by changing over from pumping to suction operation.

2. Apparatus according to claim 1 to, characterised in that the analysing device (11) has at both ends a conical socket connection (30, 30'), by which the analysing device (11) is connected on the one hand to the subcutaneous needle (2) and on the other hand to a connecting pipe (8) to the pumping and suction device (7), the socket connection (30) providing through releasable contacts (35) an electric connection (34) between the evaluating unit (18) and analysing device (11).

3. Apparatus according to claim 1 or 2, characterised in that the pumping and suction device (7) communicates in a third position of the multi-way valve (15) with an opening (16) for the supply of atmospheric air.

4. Apparatus according to one of claims 1 to 3, characterised in that the pumping and suction device (7) has a piston pump (5) as well as that an energy-storage device (14) charged during the pumping stroke is provided for the suction stroke.

5. Apparatus according to claim 4 characterised in that there is provided in the pump drive a mechanism which, between the pumping and suction stroke, in the equilibrating phase of the perfusion fluid, takes care of small pumping and suction movements of the piston (13) of the piston pump (5).

6. Apparatus according to claim 3, characterised in that at least one further container (24) for a medicament to be administered is provided as well as that the pumping and suction device communicates in at least one further position of the multi-way valve (15) with a respective one container (24) for a medicament.

7. Apparatus according to claim 6, characterised in that a container (24) for a rapidly acting insulin, a container for a slowly acting insulin and a container for a blood-sugar increasing hormone are provided.

8. Apparatus according to claim 6 or 7, characterised in that the perfusion reservoir (23) and the container (24) for medicament are easily deformable sachets of plastics and form together with the multi-way valve (15) and the associated connecting pipes (8, 26) an exchangeable unit (25).

9. Apparatus according to claim 8, characterised in that the exchangeable unit (25) is in communication through a pressure-tight connection, preferably through a penetrable septum (27) or an O-ring seal (38), with the pumping and suction device (7), the interior of the portion (26) of the connecting pipe (8) lying between the multi-way valve (15) and the pressure-tight connection (27; 38) corresponding in volume to the volume of at least one suction stroke.

10. Apparatus according to claim 9, characterised in that the exchangeable unit (25) has a diaphragm (43) which is elastically deformable by the pressure on it between the pressure-tight connection (27; 38) and the multi-way valve (15).

11. Apparatus according to claim 2, characterised in that a deformable connecting pipe (33) and an electric connection (34) are provided between the conical socket connection (30) to the multi-way valve (15) and the analysing device (11).

12. Apparatus according to claim 6, characterised in that a collecting container (37) for the perfusion fluid is provided, communicating with the pumping and suction device (7) in a further position of the multi-way valve (15).

13. Apparatus according to one of claim 1 to 3, characterised in that the pumping and suction device (7) is a precision pump (20) controlled by a microprocessor (22).

14. Apparatus according to claim 13, characterised in that the precision pump (20) has a pumping capacity of 10 to 200 µl/s and in suction operation produces a negative pressure of 200 to 700 mbar within one second.

15. Apparatus according to claim 13 or 14, characterised in that for the rapid reversal of the direction of flow of the perfusion fluid a pump space (41) exposed to negative pressure during the piping operation of the precision pump (20) is connectable in suction operation to the subcutaneous needle (2) through a connecting pipe (40) in a further position of the valve (15).

16. Apparatus according to claim 1, characterised in that a ion sensor or conductivity sensor (31) as well as a glucose sensor (32) are provided in the analysing device (11).

17. Apparatus according to claim 1, characterised in that a pre-amplifier (36) is incorporated in the analysing device (11).

18. Apparatus according to claim 1, characterised in that the analysing device (11) has on its input side a change in the cross-section for flow in the form of a projection (39).

19. Apparatus according to one of claims 16 to 19, characterised in that the analysing device (11) with the combined sensors (31, 32) has a filling volume of about 10 µl.

## Revendications

1. Dispositif pour déterminer la concentration d'au moins une substance présente dans le tissu organique, de préférence la concentration en glucose avec une aiguille sous-cutanée (2) qu'on peut insérer dans le tissu, en ayant une pompe et un dispositif d'aspiration (7) pour introduire un liquide de perfusion et pour le prélever après sa mise en équilibre partielle avec le tissu, la taux d'échange avec le tissu pouvant être déterminé par des grandeurs de marquage endogènes ou oxogènes du liquide de perfusion, ainsi qu'avec un dispositif d'analyse (11) avec des dispositifs de mesure pour saisir la concentration de la substance à mesurer et la concentration des grandeurs de marquage, caractérisé en ce qu'on prévoit une aiguille sous-cutanée (2) à un conduit dont le conduit (10) est connectable à l'entrée du dispositif d'analyse (11), que l'aiguille sous-cutanée (2) présente à côté de l'orifice frontal (2') au moins un autre orifice (3) ou un élargissement en forme de fente (42) dans le domaine de paroi, que le dispositif d'analyse (11) est fermé du côté de la sortie par un distributeur multi-voies (15) au dispositif de pompe et d'aspiration (7), de sorte que le dispositif de pompe et d'aspiration (7) dans une première position du distributeur (15) soit relié à l'aiguille sous-cutanée (2) et que dans une deuxième position, il soit relié à un récipient de perfusion (23), et que le sens d'écoulement du liquide de perfusion dans le conduit (10) de l'aiguille (2) sous-cutanée peut être inversé par changement du fonctionnement de pompage en aspiration.

2. Dispositif selon la revendication 1, caractérisé en ce que le dispositif d'analyse (11) présente de chaque côté une connexion par insertion conique (30, 30'), grâce à laquelle le dispositif d'analyse (11) est relié d'un côté à l'aiguille sous-cutanée (2) et de l'autre côté par une conduite de liaison (8) au dispositif de pompe et d'aspiration (7), la connexion d'insertion (30) établissant par des contacts (35) démontables, une liaison électrique (34) entre l'unité d'évaluation (18) et le dispositif d'analyse (11).

3. Dispositif salon la revendication 1 ou 2, caractérisé en ce que le dispositif de pompe et d'aspiration (7) dans une troisième position du distributeur multi-voies (15) est en liaison avec un orifice (16) pour l'introduction d'air extérieur.

4. Dispositif selon l'une des revendications 1 à 3, caractérisé en ce que le dispositif de pompe et d'aspiration (7) présente une pompe à piston (5), ainsi que pour la course aspirante, on a prévu un réservoir d'énergie (14) chargé pendant la course de pompage.

5. Dispositif selon la revendication 4, caractérisé en ce que dans l'entraînement de pompe, on a ménagé un mécanisme, qui entre course de pompage et course d'aspiration, dans la phase d'équilibre du liquide de perfusion, prend en charge les petits mouvements de pompage et d'aspiration du piston (13) de la pompe à piston (5).

6. Dispositif selon la revendication 3, caractérisé en ce qu'on prévoit au moins un autre récipient (24) pour administrer un médicament, et que le le dispositif de pompe et d'aspiration, dans une autre position du distributeur multi-voies (15) en communication avec un récipient (24) pour un médicament.

7. Dispositif selon la revendication 6, caractérisé en ce qu'on prévoit un récipient (24) pour une insuline rapidement efficace, un récipient pour de l'insuline efficace à long terme et un récipient pour l'hormone augmentant le taux de glucose.

8. Dispositif selon la revendication 6 ou 7, caractérisé en ce que le récipient de perfusion (23) et les récipients (24) pour des médicaments sont des poches facilement déformables en matière plastique et forment une unité échangeable (25) avec le distributeur multi-voies (15) et en les conduites de liaison correspondantes (8, 26).

9. Dispositif selon la revendication 8, caractérisé en ce que l'unité échangeable (25) est en communication étanche à la pression, de préférence par l'intermédiaire d'un tampon à perforer (27) ou d'un joint torique (38) avec le dispositif de pompe et d'aspiration (7), le volume interne de la partie de conduite (26) de la conduite de liaison (8) située entre le distributeur multi-voies (15) et la connexion étanche à la pression (27, 28) correspond au volume d'au moins un course d'aspiration.

10. Dispositif selon la revendication 9, caractérisé en ce que l'unité échangeable (25) entre la connexion étanche à la pression (27, 38) et le distributeur multi-voies (15) présente une membrane (43) élastique déformable sous la pression appliquée.

11. Dispositif selon la revendication 2, caractérisé en ce qu'entre la connexion d'insertion conique (30) vers le distributeur multi-voies (15) et le dispositif d'analyse (11), on prévoit une conduite de liaison déformable (33) et une liaison électrique (34).

12. Dispositif selon la revendications 6, caractérisé en ce qu'on prévoit un réservoir (37) pour le liquide de perfusion, qui est en communication par une autre position du distributeur multi-voies (15) avec le dispositif de pompe et d'aspiration (7).

13. Dispositif selon l'une des revendications 1 à 3, caractérisé en ce que le dispositif de pompe et d'aspiration (7) est une pompe de précision (20) commandée par un microprocesseur (22).

14. Dispositif selon la revendication 13, caractérisé en ce que la pompe de précision (20) présente une capacité de 10 à 200 µl/s et qu'en fonctionnement d'aspiration, elle produit une dépression de 200 à 700 mbars en une seconde.

15. Dispositif selon la revendication 13 ou 14, caractérisé en ce que, pour inverser rapidement le sens d'écoulement du liquide de perfusion, on peut relier un volume de pompe (41) soumis à dépression pendant le fonctionnement en pompage de la pompe de précision (20) par connexion en une autre position du distributeur (15) pour le fonctionnement en aspiration, par l'intermédiaire d'une conduite de connexion (40), à l'aiguille sous-cutanée (2).

16. Dispositif selon la revendication 1, caractérisé en ce qu'on prévoit dans le dispositif d'analyse (11) une sonde ionique ou de conductibilité (31) ainsi qu'une sonde de glucose (32).

17. Dispositif selon la revendication 1, caractérisé en ce qu'on monte un préamplificateur (36) dans le dispositif d'analyse (11).

18. Dispositif selon la revendication 1, caractérisé en ce que le dispositif d'analyse (11) présente du côté de l'entrée, une variation de section d'écoulement sous forme d'une nappe (39).

19. Dispositif selon l'une des revendications 16 à 19, caractérisé en ce que le dispositif d'analyse (11), avec les sondes associées (31, 32) présente un volume de remplissage d'environ 10 µl.
